# EUROPEAN PATENT SPECIFICATION

(11) **EP 0 357 630 B1**
(45) Date of publication and mention of the grant of the patent: **24.05.1995**
(21) Application number: 88903106.8
(22) Date of filing: 23.03.1988
(51) Int. Cl.: A61K 7/06

(54) **COMBINATION OF MINOXIDIL AND AN ANTI-INFLAMMATORY AGENT FOR TREATING PATTERNED ALOPECIA**
KOMBINATION AUS MINOXIDIL UND EINER ANTIINFLAMMATORISCHEN SUBSTANZ ZUR BEHANDLUNG DER ALOPEZIE
COMBINAISON DE MINOXYDIL ET D'UN AGENT ANTI-INFLAMMATOIRE POUR LE TRAITEMENT DE L'ALOPECIE TYPIQUE DES PERSONNES DE SEXE MASCULIN

(30) Priority: 30.03.1987 US 32362; 29.05.1987 US 55674
(43) Date of publication of application: 14.03.1990
(73) Proprietor: THE UPJOHN COMPANY, Kalamazoo, Michigan 49001 (US)
(72) Inventor: KLIGMAN, Albert, M., Philadelphia, PA 19106 (US)
(74) Representative: Perry, Robert Edward
(86) International application number: US8800870
(87) International publication number: WO8807361

(56) References cited:
- EP-A- 0 220 118
- US-A- 4 141 976
- S.T.N., File Supplier, (Karlsruhe, DE), & Chemical Abstracts, vol. 106, 5 June1987, abstract no. 201544m & JP,A, 6233111 (MURAI, TADAYOSHI) 13 February 1987
- Patent Abstracts of Japan, volume 11, no. 115 (C-415)(2562), 10 April 1987; & JP-A-61260010 (Yamaguchi Yakuhin Shokai K.K.) 18.11.1986
- Patent Abstracts of Japan, vol. 11, no. 3 (C-395)(2450), 7 January 1987; & JP-A-61183209 (LION CORP.) 15 August 1986

## Description

### BACKGROUND

The present invention relates to methods, and compositions for treating human patterned alopecia, also called androgenic alopecia involving the use of minoxidil (or related compounds) and anti-inflammatory agents.

Dermatologists recognize many different types of hair loss, the most common by far being "androgenic alopecia" wherein human males begin losing scalp hair at the temples and on the crown of the head in early adult life. This type of hair loss is more common and more severe in males, hence its common name "male pattern baldness". However, similar patterned baldness occurs in women, though it progresses more slowly and does not reach the end stage of complete denudation. An effective treatment for these and related conditions has long been sought.

There are two types of hair follicles which produce either "terminal hairs" or "vellus hairs". Terminal hairs are coarse, pigmented, long hairs in which the bulb of the follicle is situated deep in the skin, usually in the subcutaneous tissue. Vellus hairs, on the other hand, are fine, thin, non-pigmented short hairs whose bulbs are located superficially in the upper dermis. In patterned alopecia, follicles which produce terminal hairs are gradually converted to vellus ones through a miniaturization process.

Along with this progressive involution there inevitably occurs changes in the proportion of hairs in the various phases of the hair cycle. All follicles pass through a life cycle that includes three phases, namely, (1) anagen (2) catagen and (3) telogen. The anagen phase is the period of active hair growth and, on the scalp generally lasts from 3-6 years. Catagen is a short transitional phase when the follicle contracts in preparation for a resting period. It lasts a couple of weeks. In telogen, the follicle is in a resting phase where all growth ceases and the hair becomes short "club" hairs. When a new cycle begins, the club hair is shed. Telogen scalp hairs are relatively short-lived, about three to four months.

Normally, approximately 90% of scalp hairs are in the anagen phase, less than 1% in catagen and the remainder in telogen. With the onset of patterned baldness, a successively greater proportion of hairs are in the telogen phase, with correspondingly fewer in the active growth anagen phase.

Additionally, there may be some actual loss of hair follicles but this is limited to the last final phase. For the most part, the visible diminution in the bulk of hair is due to the miniaturization of the follicles. In completely bald areas, all the follicles are in the vellus phase producing ugly fine, short, non-pigmented hairs which are cosmetically useless. It may take 20 to 30 years for the distinctly anagen follicles on the crown to become transformed into a uniform population of vellus follicles.

Patterned baldness is sometimes called androgenic alopecia because male hormones are necessary for its development. It does not occur before adolescence, nor in castrates. Attempts to prevent alopecia by hormonal treatments by using anti-androgens or female hormones have failed. A hereditary component is also recognized since patterned alopecia runs in families. Despite intensive investigation, the mechanism whereby terminal follicles convert to vellus ones is unknown.

At the present time, one effective treatment for patterned alopecia is hair transplantation. Plugs of hair-bearing skin from the back of the scalp are transplanted into the bald areas. The procedure is costly and painful. Hundreds of plugs must be transplanted to create an appearance of hairiness. It is impossible to obtain anything near the original density of terminal hair.

Many other approaches for creating or reversing patterned alopecia have been tried including ultra-violet radiation, massage, chemical irritation and innumerable natural products and herbs. None of these has been generally accepted as effective.

Drugs offer a more rational approach - though for the most part have been designed to interfere with drug metabolism. The results have been poor to date. In Europe, a schedule of estrogens and antiandrogens have been administered orally to balding females with inconsistent results and with obvious limitations.

The androgenic hormone testosterone has been shown to stimulate hair growth when injected into the beard and pubic regions of adult females. Also, topical application to the armpit causes increased hair growth in aged persons. Nonetheless, topical application of testosterone has not been able to grow hair in balding individuals. Indeed, high doses of oral testosterone can induce patterned alopecia.

The topical application of minoxidil, i.e. 6-(1-piperidinyl)-2,4-pyrimidinediamine-3-oxide, is currently the most effective therapy for patterned alopecia. Minoxidil is a well-known pharmaceutical agent marketed by The Upjohn Company in the form of LONITEN® tablets for the treatment of hypertension. Numerous investigators have demonstrated that it can stimulate visible hair growth in a majority of balding subjects. The structure and use of minoxidil and related 6-amino-4-(substituted amino) -1,2-dihydro-1-hydroxy-2-iminopyrimidines are described in US-A-4139619 and US-A-4596812.

The use of retinoids alone or in combination with minoxidil and related substituted pyrimidines to increase hair growth is disclosed in WO-A-8504577, WO-A-8302558 and WO-A-8202833.

The use of minoxidil sulfate, i.e. 2,6-diamino-4-(1-piperidinyl)-1-(sulfooxy)pyrimidinium hydroxide, inner salt, as a therapeutic agent to stimulate hair growth is disclosed in WO-A-8600073.

Topical anti-inflammatory agents, such as hydrocortisone, are known, see, e.g. The Merck Index, Tenth Edition, 4689 (1983).

JP-B-61260010 states that topical formulations for baldness treatment may be prepared by mixing minoxidil with one or more of amino-acids, bactericides, anti-inflammatory agents, adrenal hormones, anti-histamines, vitamin E derivatives, estrogens and capillary vessel dilators.

According to a first aspect of the present invention, minoxidil or another, analogous compound of the formula given in claim 1, or a pharmacologically-acceptable acid addition salt thereof, and a steroidal anti-inflammatory compound, are used for the manufacture of a medicament comprising 0.1-10% w/v, preferably 1-5% w/v, of minoxidil or analogue and a major amount of a carrier or vehicle adapted for topical application, for use in treating humans for patterned alopecia.

According to a second aspect of the present invention, a medicament comprises 0.1-10% w/v, preferably 1-5% w/v, of minoxidil or an analogue of the formula defined in claim 1, or a pharmacologically-acceptable acid addition salt thereof, hydrocortisone, and a major amount of a carrier or vehicle adapted for topical application. The amount of hydrocortisone is preferably 0.5-2.5% w/v. More preferably, a medicament comprises 2% w/v of the minoxidil or analogue and 1% w/v hydrocortisone, or 3% w/v of the former and 2.5% w/v of the latter.

The present invention is based on the discovery that there is a chronic inflammatory process, subtending to the hair bulbs, in patterned alopecia, leading to eventual scarring of the lower follicle, making regrowth impossible. The present invention solves this problem by suppressing sub-bulbar inflammation, thus allowing for regrowth.

While the steroidal anti-inflammatory compound used in the first aspect of the invention is preferably hydrocortisone, it may be any steroidal compound, e.g. a corticosteroid, known to reduce inflammation by oral or topical administration.

A medicament used in the present invention may be in the form of a conventional pharmaceutical preparation such as an ointment, lotion, paste, jelly, gel, mousse, spray, foam or aerosol. The term "ointment" embraces formulations which include creams which are either oil-in-water or water-in-oil emulsions. The compounds may also be formulated into liposomal preparations or lipid emulsions or dissolved in conventional solvents such as alcohol or propanol.

The medicament is applied on a regular basis, with or without occlusion, for a period of time sufficient to effect hair growth. Occlusion of the preparation may be obtained by any conventional means such as bandages, plastic coverings, shower caps or swimming caps. The percentage of active ingredients as well as frequency of application may be varied as necessary or desirable to achieve the desired results.

The present invention is seen more by fully the examples given below.

### Example 1

Fifteen males with early alopecia were treated with a combination of 2% minoxidil and 1% hydrocortisone in a hydroalcoholic vehicle. The subjects were shampooed weekly and hairs were counted after filtration. Fewer hairs were observed to be shed as treatment progressed. Hair regrowth has been substantially better than with minoxidil alone in comparable cases. The longest period of treatment is 6 months. Hair loss has virtually stopped in all subjects.

### Example 2

Twenty-six young-adult, balding white males were treated for at least five months with a hydroalcoholic solution containing: (1) 3% minoxidil; and (2) 2.5% hydrocortisone. These subjects were in an early stage of baldness, not exceeding 51 mm (2 in) in diameter over the vertex.

The combination formulation stimulated hair growth in these subjects within three months. Most of the subjects volunteered comments of surprise that enhancement could be seen as early as two months. These results were compared with the results of a study at the same clinic of 50 patients using 3% minoxidil alone. In the opinion of the investigator, hair growth was seen sooner and on a greater proportion of patients in the group treated with the combination of minoxidil and hydrocortisone.

## Claims

1. Use of a first compound of the formula 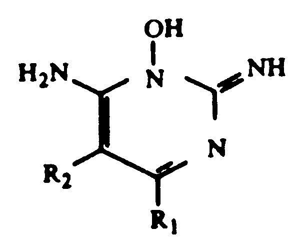 wherein R₁ is NR₃R₄ wherein R₃ and R₄ are selected from hydrogen, lower alkyl, lower alkenyl, lower aralkyl, and lower cycloalkyl, and taken together R₃ and R₄ may be a heterocyclic moiety selected from the group consisting of aziridinyl, azetidinyl, pyrrolidinyl, piperidino, hexahydroazepinyl, heptamethylenimino, octamethylenimino, morpholino and 4-lower-alkyl-piperazinyl, each of said heterocyclic moieties having attached as substituents on the carbon atoms 0-3 lower alkyl groups, hydroxy or alkoxy, and wherein R₂ is selected from hydrogen, lower alkyl, lower alkenyl, lower alkoxyalkyl, lower cycloalkyl, lower aryl, lower aralkyl, lower alkaryl, lower alkaralkyl, lower alkoxyaralkyl and lower haloaralkyl, and the pharmacologically-acceptable acid addition salts thereof, and a second compound that is a steroidal anti-inflammatory compound, for the manufacture of a medicament comprising 0.1 to 10% w/v of the first compound and a major amount of a carrier or vehicle adapted for topical application, for use in treating humans for patterned alopecia.

2. Use according to claim 1, wherein the first compound is 6-(1-piperidinyl)-2,4-pyrimidinediamine-3-oxide.

3. Use according to claim 1 or claim 2, wherein the medicament comprises 1 to 5% w/v of the first compound.

4. A medicament comprising 0.1 to 10% w/v of a first compound as defined in claim 1, hydrocortisone, and a major amount of a carrier or vehicle adapted for topical application.

5. A medicament according to claim 4, which comprises 0.5 to 2.5 w/v hydrocortisone.

6. A medicament according to claim 4 or claim 5, which comprises 1 to 5% w/v of the first compound.

7. A medicament according to claim 3, comprising 2% w/v of the first compound and 1% w/v hydrocortisone.

8. A medicament according to claim 3, comprising 3% w/v of the first compound and 2.5% w/v hydrocortisone.

9. A medicament according to any of claims 4 to 8, wherein the first compound is 6-(1-piperidinyl)-2,4-pyrimidinediamine-3-oxide.

## Patentansprüche

1. Verwendung einer ersten Verbindung der Formel: 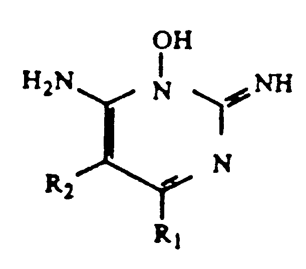 worin bedeuten:
R₁ NR₃R₄, worin R₃ und R₄ aus Wasserstoff, Niedrigalkyl, Niedrigalkenyl, Niedrigaralkyl und Niedrigcycloalkyl ausgewählt sind und R₃ und R₄ zusammen eine heterocyclische Einheit, ausgewählt aus der Gruppe Aziridinyl, Azetidinyl, Pyrrolidinyl, Piperidino, Hexahydroazepinyl, Heptamethylenimino, Octamethylenimino, Morpholino und 4-Niedrigalkylpiperazinyl, wobei jede der heterocyclischen Einheiten als Substituenten an den Kohlenstoffatomen 0 - 3 Niedrigalkylgruppen, Hydroxy oder Alkoxy aufweist, bilden können, und
R₂ Wasserstoff, Niedrigalkyl, Niedrigalkenyl, Niedrigalkoxyalkyl, Niedrigcycloalkyl, Niedrigaryl, Niedrigaralkyl, Niedrigalkaryl, Niedrigalkaralkyl, Niedrigalkoxyaralkyl und Niedrighalogenaralkyl, und von deren pharmakologisch akzeptablen Säureadditionssalzen,
und einer zweiten Verbindung, bei der es sich um eine steroide entzündungshemmende Verbindung handelt, zur Herstellung eines Medikaments mit 0,1 - 10% g/v der ersten Verbindung und eines größeren Anteils an einem zur topischen Applikation geeigneten Träger oder Vehikel
zur Verwendung bei der Behandlung von Musteralopezie bei Menschen.

2. Verwendung nach Anspruch 1, wobei die erste Verbindung aus 6-(1-Piperidinyl)-2,4-pyrimidindiamin-3-oxid besteht.

3. Verwendung nach Anspruch 1 oder 2, wobei das Medikament 1 - 5% g/v der ersten Verbindung enthält.

4. Medikament mit 0,1 - 10% g/v einer ersten Verbindung gemäß der Definition nach Anspruch 1, Hydrocortison und einem größeren Anteil eines zur topischen Anwendung geeigneten Trägers oder Vehikels.

5. Medikament nach Anspruch 4, umfassend 0,5 - 2,5 g/v Hydrocortison.

6. Medikament nach Anspruch 4 oder 5, umfassend 1 - 5% g/v der ersten Verbindung.

7. Medikament nach Anspruch 3, umfassend 2% g/v der ersten Verbindung und 1% g/v Hydrocortison.

8. Medikament nach Anspruch 3, umfassend 3% g/v der ersten Verbindung und 2,5% g/v Hydrocortison.

9. Medikament nach einem der Ansprüche 4 bis 8, wobei die erste Verbindung aus 6-(1-Piperidinyl)-2,4-pyrimidindiamin-3-oxid besteht.

## Revendications

1. Utilisation d'un premier composé de formule 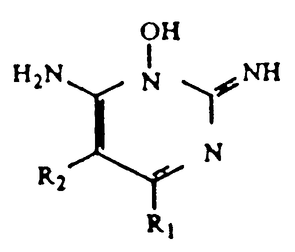 dans laquelle R₁ représente un groupe NR₃R₄ dans lequel R₃ et R₄ sont choisis entre l'hydrogène, des groupes alkyle inférieur, alcényle inférieur, aralkyle inférieur et cycloalkyle inférieur, les groupes R₃ et R₄, pris conjointement, pouvant représenter un groupe hétérocyclique choisi entre des groupes aziridinyle, azétidinyle, pyrrolidinyle, pipéridino, hexahydroazépinyle, heptaméthylèneimino, octaméthylèneimino, morpholino et 4-(alkyle inférieur)-pipérazinyle, chacun de ces groupements hétérocycliques portant comme substituants sur les atomes de carbone 0 à 3 groupes alkyle inférieur, hydroxy ou alkoxy, et dans laquelle R₂ est choisi entre l'hydrogène, des groupes alkyle inférieur, alcényle inférieur, alkoxyalkyle inférieur, cycloalkyle inférieur, aryle inférieur, aralkyle inférieur, alkaryle inférieur, alkaralkyle inférieur, alkoxyaralkyle inférieur et halogénaralkyle inférieur, et de ses sels d'addition d'acides pharmacologiquement acceptables, et d'un second composé qui est un composé anti-inflammatoire stéroïdien, pour la production d'un médicament comprenant 0,1 à 10 % en poids/volume du premier composé et une quantité dominante d'un support ou véhicule apte à une application locale, dans le traitement de sujets humains atteints de calvitie hippocratique.

2. Utilisation suivant la revendication 1, dans laquelle le premier composé est le 3-oxyde de 6-(1-pipéridinyl)-2,4-pyrimidinediamine.

3. Utilisation suivant la revendication 1 ou la revendication 2, dans laquelle le médicament comprend 1 à 5 % en poids/volume du premier composé.

4. Médicament comprenant 0,1 à 10 % en poids/volume d'un premier composé répondant à la définition suivant la revendication 1, de l'hydrocortisone et une quantité dominante d'un support ou véhicule apte à une application locale.

5. Médicament suivant la revendication 4, qui comprend 0,5 à 2,5 % en poids/volume d'hydrocortisone.

6. Médicament suivant la revendication 4 ou la revendication 5, qui comprend 1 à 5 % en poids/volume du premier composé.

7. Médicament suivant la revendication 3, comprenant 2 % en poids/volume du premier composé et 1 % en poids/volume d'hydrocortisone.

8. Médicament suivant la revendication 3, comprenant 3 % en poids/volume du premier composé et 2,5 % en poids/volume d'hydrocortisone.

9. Médicament suivant l'une quelconque des revendications 4 à 8, dans lequel le premier composé est le 3-oxyde de 6-(1-pipéridinyl)-2,4-pyrimidinediamine.
